Europäisches Patentamt

European Patent Office

Office européen des brevets

(19)

(11) Publication number: **0 088 621**
A2

# EUROPEAN PATENT APPLICATION

(21) Application number: 83301206.5

(22) Date of filing: 07.03.83

(51) Int. Cl.³: **A 61 K 31/70**, A 61 K 31/405,
A 61 K 31/195
// (A61K31/70, 31/405, 31/195)

(30) Priority: 08.03.82 US 355967

(43) Date of publication of application: 14.09.83
Bulletin 83/37

(84) Designated Contracting States: **AT BE CH DE FR GB IT
LI LU NL SE**

(71) Applicant: **MASSACHUSETTS INSTITUTE OF
TECHNOLOGY**, 77 Massachusetts Avenue, Cambridge
Massachusetts 02139 (US)

(72) Inventor: **Wurtman, Richard J.**, 193 Marlborough Street,
Boston Massachusetts 02116 (US)

(74) Representative: **Holdcroft, James Gerald, Dr. et al,**
Graham Watt & Co. Riverhead, Sevenoaks Kent
TN13 2BN (GB)

(54) **Compositions for use in accelerating onset of sleep.**

(57) A composition for use in accelerating onset of sleep comprises an insulin releasing carbohydrate, and tryptophan. The weight ratio of carbohydrate (as glucose equivalent) to tryptophan is between 8 and 20. The composition may also contain tyrosine to maintain or increase brain dopamine and/or norepinephrine levels, which can be important where the patient also suffers from e.g. hypertension or depression.

EP 0 088 621 A2

ACTORUM AG

- 1 -

# COMPOSITIONS FOR USE IN ACCELERATING

## ONSET OF SLEEP

This invention relates to accelerating sleep onset.

Presently, sleep onset is accelerated and sleep quality improved by administering certain drugs. Commonly, barbiturate or benzodiazepine drugs like pentobarbital or Dalmane, (a Trademark of Hoffmann La Roche), have been administered orally and have been found effective in accelerating sleep onset. However, each of these drugs has unwanted side effects (such as drowsiness the next day, or potentiation of the consequences of drinking alcoholic beverages) and in some instances can be dangerous to the user. In addition, the generally-used sleep promoting drugs often interfere with important aspects of sleep, - for example, suppressing REM sleep and dreaming. This feature can make them disadvantageous, especially for long-term use.

It is an object of the present invention to provide means for accelerating sleep onset and improving sleep quality in a selective manner without producing the undesirable side effects caused by barbiturates or benzodiazepines.

- 2 -

It has been found that a combination of tryptophan and carbohydrate (which causes insulin secretion) can selectively accelerate sleep onset and improve sleep quality. The mixture of tryptophan and an insulin-secreting carbohydrate can be administered alone or in admixture with tyrosine. It is believed that these compositions function by mechanisms which involve the enhancement of brain serotonin synthesis; this neurotransmitter is involved in the onset of sleep, and probably also in determining sleep quality. Tyrosine is a precursor for dopamine and norepinephrine in synapses; its co-administration permits serotonin synthesis to be accelerated while not reducing the synthesis of dopamine and/or norepinephrine. In some situations, such as in people with hypertension or depression, it is important not to lower brain dopamine and/or norepinephrine synthesis; moreover brain norepinephrine may also be important for optimal sleep quality.

In European Patent Application No. 7691 of Massachusetts Institute of Technology, compositions are described for decreasing appetite which comprise tryptophan and a carbohydrate. The entire disclosure of Application No. 7691 is incorporated herein by reference including the contents of the official file as well as the printed Patent Application.

Surprisingly it has now been found that compositions containing tryptophan and a carbohydrate are effective for accelerating onset of sleep, particularly, but not exclusively when the weight ratio of carbohydrate (as glucose equivalent) to tryptophan is between 8 and 20.

The present invention provides a composition for use in accelerating onset of sleep characterised in that it comprises an insulin releasing carbohydrate, and tryptophan.

The invention also provides a composition which, when administered to an animal, accelerates onset of sleep characterised in that it consists essentially of an amount of tryptophan effective to increase brain serotonin levels and a carbohydrate in an amount effective to cause insulin to be released in the animal; and the weight ratio of carbohydrate (as glucose equivalent) to tryptophan is between 8 and 20, e.g. between 8 and 16.

The invention also provides a composition according to the invention comprising tyrosine in an amount effective to maintain or increase brain dopamine and/or norepinephrine levels.

- 4 -

The invention also provides a composition according to the invention also comprising an excipient.

The invention also provides a composition according to the invention in pharmaceutical dosage form.

It will be realized for example that a composition according to the invention may be provided with the tryptophan and carbohydrate in admixture or alternatively as a two-part composition with a part A comprising the tryptophan and a part B comprising the carbohydrate.

There now follows a description of embodiments of the invention. This description is given by way of example only and not by way of limitation of the invention.

In embodiments of the invention tryptophan and an insulin-releasing carbohydrate are administered to a patient either alone or in combination with tyrosine. The administration of tryptophan changes the ratio of tryptophan to the sum of plasma concentrations of other neutral amino acids that compete with tryptophan for uptake in the brain, thereby increasing the brain serotonin level. Furthermore, the administration of a carbohydrate that releases insulin decreases the plasma levels of the other large neutral amino acids (LNAA) normally found in the plasma such as leucine, isoleucine, tyrosine, phenylalanine and valine. Thus, the carbohydrate causes an increase of the plasma levels of tryptophan in relation to these other amino acids in the plasma. Both of these effects are cumulative in effecting an increase in brain serotonin levels.

Representative suitable carbohydrates include sucrose, dextrose, starch, fructose, invert sugar, dextrins, sugar polymers such as polyose, xylitol and mixtures thereof.

The weight ratio of the tryptophan to the carbohydrate (as glucose equivalent) is between 1:8 and 1:20, more usually between 1:8 and 1:16. The compositions are administered in an amount sufficient to effect increase in brain serotonin levels while not being administered in such large amounts as to seriously reduce the brain levels of other neurotransmitters needed for normal functioning such as dopamine, norepinephrine, acetylcholine or the non-essential amino acids. Generally, the compositions are administered in an amount of between 3 mg/kg and 30 mg/kg of tryptophan, and 25 mg/kg and 250 mg/kg of carbohydrate (as glucose equivalent), more usually between 5 mg/kg and 25 mg/kg of tryptophan and 50-100 mg/kg of carbohydrate. Typical unit dosage found useful for oral administration ranges between 0.25 grams and 25 grams, and more usually between 1 gram and 10 grams. When tyrosine is administered, it is given in an amount of between 3 mg/kg and 50 mg/kg and at a weight ratio of tryptophan to tyrosine of between 0.3 and 3.0, preferably between 0.5 and 2.0.

The tryptophan alone or with tyrosine can be administered as free amino acids, esters, salts, neutral or synthetic polymers or as constituents of food. The route of administration will generally be oral, for example, as a tablet, sustained-release capsule, drink, beverage sweetener, wafer, candy or chewing gum.

Administration of the present combination of tryptophan and an insulin-secreting carbohydrate in an amount effective to stimulate insulin secretion provides substantial advantages over the administration of tryptophan alone. The dosages of tryptophan alone needed to produce sleepiness (e.g., 3-4 grams/day) can be inconvenient to administer, and can produce undesirable side effects such as gastrointestinal upset. Moreover they can cause the formation of excessive amounts of metabolites which may be related to bladder neoplasias, and can, by competing with tyrosine for

- 7 -

brain uptake, impair brain catecholamine, synthesis.

EXAMPLE I

Normal adult volunteers consumed 1 g of tryptophan; with or without 10 g of glucose; blood samples were assayed for tryptophan, LNAA, and their ratio after various intervals. Control subjects received only the tryptophan. The results are shown in Table I, 1 hour after administration.

TABLE I

Plasma Tryptophan/LNAA Ratios

| Dosage | 0.5 g try | 1.0 g try | 0.5 g try/ 10 g glucose | 1.0 g try/ 10 g glucose |
|--------|-----------|-----------|----------------|----------------|
| | 0.20 | 0.31 | 0.29 | 0.43 |

As shown in Table I, the glucose had the effect of increasing the ratio by about 40-50%; this would be expected to cause a parallel effect on brain tryptophan level and on brain serotonin synthesis.

EXAMPLE II

Healthy adult subjects with no history of frequent sleep disturbance (but with occasional difficulty in falling asleep right away), and who usually

- 8 -

went to bed around 11 p.m., were asked to take a single 500 mg capsule or pill containing L-tryptophan with a beverage that either contained no carbohydrate or was fortified with 10 grams of glucose or its equivalent. They were asked to provide the following information (using a scale of 1-10, with 10 maximal):

1. Sleepiness at 10 p.m.

2. Sleepiness at 10.30 p.m.

3. Sleepiness at 11.00 p.m.

4. Estimate of the amount of time that passed between going to bed and falling asleep.

5. Estimate (in the morning) of the quality of the sleep.

6. Number of times (subjective) awakened during the night.

7. Presence or absence of (subjective) abnormal dream patterns.

8. Whether they felt rested on the following day.

Uniformly subjects reported that they felt sleepier at bedtime (11 p.m.) when they took both tryptophan plus carbohydrate than when they took tryptophan alone (or, on other nights, took nothing). They described this as a desirable feeling. One subject - who awakened several times during both evenings because of a mild unrelated pain - stated that she was aware of being very sleepy when awakened, and fell right back to sleep each time, on the evening that she had taken tryptophan plus carbohydrate. Sleep quality-estimated the following morning - was good after either treatment, and subjects did not associate changes in dream patterns with either treatment.

0088621

- 10 -

CLAIMS: (Except for Austria)

1. A composition for use in accelerating onset of sleep characterised in that it comprises an insulin releasing carbohydrate, and tryptophan.

2. A composition which, when administered to an animal, accelerates onset of sleep characterised in that it consists essentially of an amount of tryptophan effective to increase brain serotonin levels and a carbohydrate in an amount effective to cause insulin to be released in the animal; and the weight ratio of carbohydrate (as glucose equivalent) to tryptophan is between 8 and 20, e.g. between 8 and 16.

3. A composition according to claim 1 or claim 2, wherein the carbohydrate is a sugar e.g. sucrose or glucose.

4. A composition according to any one of claims 1, 2 and 3, suitable for administration in a dosage of between 3 mg/kg and 30 mg/kg (e.g. between 5 and 25) of tryptophan and between 25 mg/kg and 250 mg/kg (e.g. between 50 and 100) of carbohydrate (as glucose equivalent).

5. A composition according to any one of the preceding claims, comprising tyrosine in an amount effective to maintain or increase brain dopamine and/or norepinephrine levels.

0088621

- 11 -

6. A composition according to claim 5, wherein the weight ratio of tryptophan to tyrosine is between 0.3 and 3.0 e.g. between 0.5 and 2.0.

7. A composition according to claim 5 or claim 6, suitable for administration in a dosage of between 3 mg/kg and 50 mg/kg of tyrosine.

8. A composition according to any one of the preceding claims, also comprising an excipient.

9. A composition according to any one of claims 1 to 7, in pharmaceutical dosage form.

10. A composition according to any one of claims 1 to 7, in unit dosage form for example of between 0.25 grams and 25 grams per unit, e.g. between 1 gram and 10 grams.

11. A composition according to any one of the preceding claims, wherein the components of the composition are present in admixture.

CLAIMS: (for Austria)

1. A method of manufacturing a composition for use in accelerating onset of sleep characterised in that an insulin releasing carbohydrate is mixed with tryptophan.

2. A method of manufacturing a composition which, when administered to an animal, accelerates onset of sleep characterised in that an amount of tryptophan effective to increase brain serotonin levels is mixed with a carbohydrate in an amount effective to cause insulin to be released in the animal; and the weight ratio of carbohydrate (as glucose equivalent) to tryptophan in the composition is between 8 and 20, e.g. between 8 and 16.

3. A method according to claim 1 or claim 2, wherein the carbohydrate is a sugar e.g. sucrose or glucose.

4. A method according to any one of claims 1, 2 and 3, wherein the composition is manufactured in a form suitable for administration in a dosage of between 3 mg/kg and 30 mg/kg (e.g. between 5 and 25) of tryptophan and between 25 mg/kg and 250 mg/kg (e.g. between 50 and 100) of carbohydrate (as glucose equivalent).

5. A method according to any one of the preceding claims, wherein tyrosine is included in the composition in an amount effective to maintain or increase brain dopamine and/or norepinephrine levels.

6. A method according to claim 5, wherein the weight ratio of tryptophan to tyrosine in the composition is between 0.3 and 3.0 e.g. between 0.5 and 2.0.

7. A method according to claim 5 or claim 6, wherein the composition is manufactured in a form suitable for administration in a dosage of between 3 mg/kg and 50 mg/kg of tyrosine.

8. A method according to any one of the preceding claims, wherein an excipient is also included in the composition.

9. A method according to any one of claims 1 to 7, wherein the composition is manufactured in pharmaceutical dosage form.

10. A method according to any one of claims 1 to 7, wherein the composition is manufactured in unit dosage form for example of between 0.25 grams and 25 grams per unit, e.g. between 1 gram and 10 grams.